(19) Europäisches Patentamt
European Patent Office
Office européen des brevets

(11) EP 0 679 629 B1

(12) **FASCICULE DE BREVET EUROPEEN**

(45) Date de publication et mention de la délivrance du brevet:
**12.08.1998 Bulletin 1998/33**

(51) Int Cl.[6]: **C07C 59/68**, C07C 51/367, C07C 69/712, C07C 67/31

(21) Numéro de dépôt: **95400892.6**

(22) Date de dépôt: **21.04.1995**

(54) **Procédé de préparation d'un acide alpha-(hydroxyphenoxy)-alcanecarboxylique optiquement actif et de ses dérivés**

Verfahren zur Herstellung von optisch aktiver alpha-(Hydroxyphenoxy)-alkancarbonsäure und ihren Derivaten

Process for preparing optically active alpha-(hydroxy-phenoxy)alkane carboxylic acid and its derivatives

(84) Etats contractants désignés:
**CH DE FR GB IT LI**

(30) Priorité: **25.04.1994 FR 9404933**

(43) Date de publication de la demande:
**02.11.1995 Bulletin 1995/44**

(73) Titulaire: **RHODIA CHIMIE**
**92408 Courbevoie Cédex (FR)**

(72) Inventeur: **Metivier, M. Pascal**
**F-69110 - Ste Foy les Lyon (FR)**

(74) Mandataire: **Dutruc-Rosset, Marie-Claude et al**
**RHODIA CHIMIE**
**Direction de la Propriété Industrielle**
**25, quai Paul Doumer**
**92408 Courbevoie Cédex (FR)**

(56) Documents cités:
**EP-A- 0 009 285**
**EP-A- 0 082 413**
**EP-A- 0 192 849**
**DE-A- 2 854 542**
**FR-A- 2 460 286**
**FR-A- 2 486 071**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet un nouveau procédé de préparation d'un acide α-(hydroxyphénoxy)alcanecarboxylique optiquement actif et de ses dérivés.

Plus particulièrement, la présente invention concerne la préparation de l'acide D-2(4-hydroxyphénoxy)propionique optiquement pur dénommé de manière simplifiée, par l'abréviation " D-HPPA ".

L'invention vise également les dérivés des acides α-(hydroxyphénoxy)-alcanecarboxyliques, et plus particulièrement, leurs esters alkyliques.

Les esters aliphatiques de l'acide D-2(4-hydroxyphénoxy)propionique optiquement purs sont des intermédiaires d'herbicides sélectifs très recherchés actuellement.

Ces esters peuvent être obtenus directement par inversion de Walden à partir de L-chloro ou L-bromopropionate d'alkyle et d'hydroquinone, en présence d'une base forte en milieu aqueux ou alcoolique. Malheureusement, dans ces milieux, les esters sont facilement hydrolysés et le motif chloropropionique subit une racémisation, tel que décrit par W.A Cowdrey et al [Journal of Chemical Society 1937, 1208].

Ces esters peuvent être également préparés à partir de l'acide D-2(4-hydroxyphénoxy)-propionique. D-2(4-hydroxyphénoxy)propionique Or, la préparation de l'acide présente deux difficultés majeures : fabriquer un produit de haute pureté optique et obtenir un produit monosubstitué d'hydroquinone avec une sélectivité élevée.

La synthèse du D-HPPA est conduite selon les procédés décrits dans l'état de la technique, en milieu alcoolique ou en milieu aqueux.

Ainsi, la demande japonaise JP-A 62/16446 décrit la préparation du D-HPPA, par réaction du sel de sodium de l'acide L-α-chloropropionique en solution éthanolique et d'hydroquinone en présence de soude. Le procédé décrit est conduit en milieu anhydre. A cet effet, le sel de sodium de l'acide L-α-chloropropionique est obtenu à partir de l'ester méthylique de l'acide L-α-chloropropionique ajouté à une solution aqueuse d'hydroxyde de sodium puis élimination de l'eau par distillation sous pression réduite permettant d'obtenir un solide blanc qui est récupéré et dissous dans l'éthanol.

Ce procédé requiert la nécessité de travailler en milieu anhydre ce qui est très contraignant d'un point de vue industrielle et la manipulation du solide est mal aisée car il s'agit d'un produit collant.

Par ailleurs, il est connu de préparer le D-HPPA, en milieu aqueux et les deux références citées ci-après illustrent cette voie de synthèse.

Le brevet EP-B-108 374 revendique un procédé de préparation d'acides hydroxyphénoxy-alcanecarboxyliques dont l'acide 2(4-hydroxyphénoxy)propionique.

La caractéristique du procédé breveté est de mélanger à une température inférieure à 60°C, un dihydroxybenzène en solution alcaline avec un acide 2-halogénoalcanecarboxylique (ou leur halogénure ou ester), puis à faire passer le mélange en continu dans un réacteur tubulaire à des températures de 80 à 120°C.

Ce document enseigne qu'une monoalkylation est obtenue grâce au passage continu du mélange réactionnel dans un réacteur tubulaire, pendant une courte période et à une température élevée. Un tel procédé requiert un appareillage spécifique.

Il est aussi décrit dans le brevet européen EP-A 192 849, un procédé de préparation d'un dérivé d'un acide (4-hydroxy 2-phénoxy) propionique optiquement actif, qui consiste à faire réagir un sel alcalin d'un acide α-halogénopropionique optiquement actif avec le dihydroxybenzène ou un sel alcalin d'hydroquinone, en présence d'un hydroxyde de métal alcalin et d'une quantité d'eau appropriée ce qui conduit à la précipitation du sel disodique de l'acide (4-hydroxy 2-phénoxy) propionique optiquement actif. L'eau est mise en oeuvre en une quantité telle que le rapport pondéral eau/hydroquinone varie de 1 à2,5.

Il a maintenant été trouvé et c'est ce qui constitue l'objet de la présente invention un nouveau procédé de préparation d'un acide α-(hydroxyphénoxy)alcanecarboxylique optiquement actif, entre autres, de D-HPPA, qui pallie certains inconvénients par rapport aux procédés antérieurs de l'état de la technique ou qui présente certains avantages par rapport à d'autres.

Plus précisément, la présente invention a pour objet un procédé de préparation d'un acide α-(hydroxyphénoxy) alcanecarboxylique optiquement actif et de ses dérivés caractérisé par le fait qu'il consiste :

- dans une première étape, à préparer une solution d'un sel alcalin d'un acide α-halogénoalcanecarboxylique optiquement actif, par saponification en milieu alcoolique, d'un ester alkylique d'un acide α-halogénoalcanecarboxylique optiquement actif à l'aide d'un hydroxyde de métal alcalin en solution aqueuse,
- dans une deuxième étape, à engager directement la solution issue de l'étape précédente comprenant le sel alcalin d'un acide α-halogénoalcanecarboxylique dans la réaction avec un dihydroxybenzène ou un sel alcalin de dihydroxybenzène, en présence d'un hydroxyde de métal alcalin et dans un solvant alcoolique,
- dans une dernière étape, à récupérer à partir du milieu réactionnel, l'acide α-(hydroxyphénoxy)alcanecarboxylique optiquement actif ou dérivé issu de celui-ci.

Il a été trouvé de façon inattendue que l'acide α-(hydroxyphénoxy)alcanecarboxylique obtenu à partir de l'ester alkylique d'un acide α-halogénoalcanecarboxylique ne pouvait être conduit en milieu alcoolique et en continu, sans racémisation, que dans la mesure où l'on effectuait une présaponification de l'ester alkylique de l'acide α-halogénoalcanecarboxylique, avant de le mettre, en présence de le dihydroxybenzène, sous forme salifiée.

Le procédé de l'invention est conduit en milieu alcoolique ce qui présente l'avantage d'une cinétique réactionnelle plus grande d'où un gain de productivité.

De plus, il est particulièrement intéressant à mettre en oeuvre lorsque que l'on souhaite préparer les dérivés esters car la réaction d'estérification peut être enchaînée subséquemment dans l'alcool.

Le procédé est conduit sans racémisation et le rendement optique à savoir le rapport exprimé en pourcentage entre l'excès énantiomérique de racide α-(hydroxyphénoxy)alcanecarboxylique et l'excès énantiomérique de l'ester alkylique d'un acide α-halogénoalcanecarboxylique est proche de 100 %, et le plus souvent compris entre 98 et 100 %.

Le procédé de l'invention s'applique plus préférentiellement à la préparation des acides α-(hydroxyphénoxy)alcanecarboxyliques répondant à la formule générale (I) :

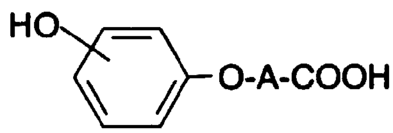

(I)

dans ladite formule (I) :

- A représente un radical méthylène éventuellement substitué par 1 ou 2 groupements alkyle ayant de 1 à 4 atomes de carbone, de préférence un groupement méthyle,
- et le groupement hydroxyle étant en position 2-, 3- ou 4- par rapport à la fonction éther.

L'acide obtenu est un produit optiquement actif : l'atome de carbone optiquement actif pris en considération étant l'atome de carbone en α du groupe COOH.

Conformément au procédé de l'invention, on commence dans une première étape, à préparer une solution d'un sel alcalin d'un acide α-halogénoalcanecarboxylique optiquement actif, par saponification en milieu alcoolique, d'un ester alkylique d'un acide α-halogénoalcanecarboxylique optiquement actif, à l'aide d'un hydroxyde de métal alcalin en solution aqueuse.

On part d'un ester alkylique d'un acide α-halogénoalcanecarboxylique répondant plus particulièrement à la formule (II) :

$$X\text{-}A\text{-}COO\ R \qquad (II)$$

dans ladite formule (II) :

- A représente un radical méthylène substitué par 1 ou 2 groupements alkyle ayant de 1 à 4 atomes de carbone, de préférence un groupement méthyle,
- R représente un groupement alkyle, linéaire ou ramifié ayant de 1 à 4 atomes de carbone,
- X représente un atome d'halogène, de préférence un atome de chlore ou de brome.

A titre d'exemples de composés répondant à la formule (II), on peut citer plus particulièrement, les esters méthylique ou éthylique des acides carboxyliques suivants sous forme D ou L:

- l'acide α-chloropropionique
- l'acide α-bromopropionique
- l'acide α-chlorobutyrique
- l'acide α-bromobutyrique
- l'acide α-chloroisobutyrique
- l'acide α-bromoisobutyrique

Parmi tous les esters d'acides précités, on met en oeuvre préférentiellement l'isomère L- ou D- de l'acide α-chloropropionique.

On prépare donc le sel alcalin d'un acide α-halogénoalcanecarboxylique optiquement actif à partir d'un ester alkylique en faisant réagir ce dernier avec un hydroxyde de métal alcalin en solution aqueuse, en milieu alcoolique.

On choisit, de préférence, la nature de l'ester mis en oeuvre de telle sorte que l'alcool libéré par saponification soit le même que l'alcool qui est utilisé comme solvant réactionnel.

On part de l'isomère optique de formule (II) ayant la configuration D- ou L-souhaitée, sachant que la réaction conduit à l'inversion de la stéréochimie de l'isomère de départ.

On choisit de préférence un composé de formule (II) ayant une bonne pureté optique, généralement moins de 10 % de l'autre énantiomère, de préférence moins de 5 %, et encore plus préférentiellement moins de 3 %.

On trouve dans le commerce des composés de formule (II) répondant aux exigences précitées et notamment l'ester méthylique de l'acide L-α-chloropropionique commercialisée par la Société RHONE-POULENC.

Comme agent de saponification, on fait appel à un agent alcalin, de préférence, l'hydroxyde de sodium ou de potassium.

L'hydroxyde de sodium est préférée.

La mise en oeuvre de l'agent alcalin peut être faite sous la forme d'une solution aqueuse ou sous la forme solide avec addition parallèle de l'eau nécessaire à sa dissolution.

La concentration de la solution aqueuse de l'hydroxyde alcalin peut varier avantageusement entre 30 et 60 % en poids. Les solutions disponibles dans le commerce conviennent tout à fait bien en particulier la solution d'hydroxyde de sodium à 36 % en poids.

La quantité de base à mettre en oeuvre est généralement en excès. Elle est déterminée de telle sorte que le rapport entre le nombre de moles d'hydroxyde de métal alcalin et le nombre de moles de l'ester alkylique de l'acide α-halogénoalcanecarboxylique varie, de préférence, entre 1,0 et 1,2.

La réaction de saponification est conduite en milieu alcoolique. L'alcool utilisé comme solvant réactionnel, est un alcanol primaire ayant de 1 à 4 atomes de carbone.

On fait appel, de préférence, au méthanol et à l'éthanol, et encore plus préférentiellement au méthanol.

On préfère également mettre en oeuvre un alcool ayant une bonne pureté chimique de préférence supérieure à 90 %.

La quantité d'alcool mise en jeu est déterminée de telle sorte qu'elle représente par rapport au poids du sel de l'acide α-halogénoalcanecarboxylique obtenu de 5 à 40 %.

La température de la réaction de saponification est choisie, de préférence entre 0°C et 40°C et plus préférentiellement entre 20°C et 25°C.

La réaction est conduite avantageusement sous pression atmosphérique.

Un mode de réalisation pratique de l'invention consiste à introduire la solution d'hydroxyde de métal alcalin dans le milieu réactionnel comprenant l'ester alkylique de l'acide α-halogénoalcanecarboxylique ou inversement. L'addition est faite de manière progressive, en continu ou en discontinu, par fractions.

La vitesse d'addition est telle que la température soit maintenue dans la zone prédéfinie car la réaction de saponification est une réaction exothermique.

On maintient le milieu réactionnel sous agitation.

En fin de saponification, on ajoute le solvant alcoolique en une quantité telle que prédéfinie. Il est également possible de l'introduire avant la saponification, avec l'ester alkylique.

Selon le procédé de l'invention, on fait réagir dans une deuxième étape, directement la solution issue de l'étape précédente comprenant le sel alcalin d'un acide α-halogénoalcanecarboxylique avec le dihydroxybenzène ou un sel alcalin de le dihydroxybenzène, en présence d'un hydroxyde de métal alcalin et dans un solvant alcoolique,

On fait appel à un dihydroxybenzène de formule (III):

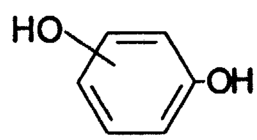

(III)

dans ladite formule (III) le groupement hydroxyle étant en position 2-, 3- ou 4- par rapport au groupement hydroxyle.

En ce qui concerne le dihydroxybenzène de formule (III), le composé préféré est l'hydroquinone.

On préfère également mettre en oeuvre un dihydroxybenzène ayant une bonne pureté chimique de préférence supérieure ou égale à 98 %.

Le dihydroxybenzène qui est mis à réagir sous une forme salifiée peut être obtenu en le mettant en présence d'un agent alcalin en même temps que le sel alcalin de l'acide α-halogénoalcanecarboxylique.

Un mode préféré consiste d'abord à salifier le dihydroxybenzène, en milieu alcoolique, en le faisant réagir avec

un agent alcalin avant de le mettre en présence avec le sel alcalin de l'acide α-halogénoalcanecarboxylique.

On fait appel à un agent alcalin, de préférence le même que celui utilisé à l'étape de saponification et encore plus préférentiellement, dans la même forme.

La quantité de base à mettre en oeuvre est généralement en excès. Elle est déterminée de telle sorte que le rapport entre le nombre de moles d'hydroxyde de métal alcalin et le nombre de moles de dihydroxybenzène varie, de préférence, entre 2,0 et 2,2.

La réaction de salification est conduite en milieu alcoolique. L'alcool utilisé est de préférence le même que celui utilisé à l'étape précédente.

La quantité d'alcool mise en jeu est déterminée de telle sorte qu'elle représente par rapport au poids du dihydroxybenzène engagé de 100 à 300 %, de préférence, environ 150 %.

La température de la réaction de salification n'est pas critique. Elle est choisie, de préférence entre 20°C et 60°C.

La réaction est conduite avantageusement sous pression atmosphérique.

On conduit la réaction de préférence sous atmosphère d'un gaz inerte qui peut être l'azote ou un gaz rare, de préférence l'argon.

D'un point de vue pratique, on introduit l'hydroxyde de métal alcalin sous forme solide ou en solution aqueuse, dans le milieu réactionnel comprenant le dihydroxybenzène et le solvant alcoolique. L'addition est faite de manière progressive, en continu ou en discontinu, par fractions.

La vitesse d'addition est telle que la température soit maintenue dans la zone prédéfinie car la réaction de salification est une réaction exothermique.

On maintient le milieu réactionnel sous agitation.

On fait ensuite réagir le dihydroxybenzène sous la forme de son disel alcalin avec la solution issue de l'étape précédente comprenant le sel alcalin d'un acide α-halogénoalcanecarboxylique.

La quantité des réactifs engagés est déterminée de telle sorte que le rapport entre le nombre de moles de dihydroxybenzène salifié et le nombre de moles du sel alcalin d'un acide α-halogénoalcanecarboxylique est compris avantageusement entre 1,0 et 1,5.

La température de la réaction est choisie, de préférence inférieure à 60°C, plus particulièrement entre 30°C et 55°C et encore plus préférentiellement entre 40°C et 45°C.

Selon un mode préféré de réalisation pratique de l'invention, on introduit progressivement la solution issue de l'étape précédente dans le milieu réactionnel comprenant le dihydroxybenzène sous forme salifiée.

Pour parfaire la réaction, on peut maintenir le milieu réactionnel sous agitation pendant une durée allant, de préférence, entre 1 et 3 heures mais cette opération n'est pas obligatoire.

Dans une dernière étape, on récupère à partir du milieu réactionnel, l'acide α-(hydroxyphénoxy)alcanecarboxylique optiquement actif, d'une manière connue en soi.

En fin de réaction, on neutralise à pH égal à environ 7, par ajout d'un acide fort tel que par exemple, l'acide chlorhydrique, l'acide sulfurique, l'acide phosphorique mis en oeuvre généralement en solution aqueuse. Sa concentration n'est pas critique.

L'acide chlorhydrique est préféré. Il est mis en oeuvre sous forme de solution aqueuse dont la concentration correspond, de préférence, à la concentration de la forme commerciale, soit 37 %.

On obtient le monosel de l'acide α-(hydroxyphénoxy)alcanecarboxylique.

On élimine le solvant alcoolique et l'alcool formé par distillation.

L'hydroquinone [ou composé de formule (III)] est extraite du milieu réactionnel par un solvant organique, de préférence, un solvant acétonique et encore plus préférentiellement, la méthylisobutylcétone ou un solvant de type éther, tel que, par exemple, le méthyl-ter-butyléther ou l'éthyl-ter-butyléther.

Après séparation des phases organique et aqueuse, la solution aqueuse résiduaire est distillée de façon à éliminer le solvant organique en solution dans la phase aqueuse puis elle est acidifiée à un pH inférieur ou égal à 1 de façon à précipiter l'acide α-(hydroxyphénoxy)alcanecarboxylique.

Une autre variante d'application de l'invention consiste à préparer les esters alkyliques ayant, de préférence de 1 à 4 atomes de carbone, des acides α-(hydroxyphénoxy)alcanecarboxyliques optiquement actifs, à partir des acides α-(hydroxyphénoxy)alcanecarboxyliques obtenus selon le procédé de l'invention, selon toute méthode connue de l'Homme du Métier.

Le procédé de l'invention permet de préparer très aisément lesdits esters. A cet effet, on neutralise la phase aqueuse à un pH inférieur ou à égal à 2,0 par ajout, comme précédemment décrit, d'un acide fort, de préférence l'acide chlorhydrique, puis on chauffe sous reflux de l'alcanol (de préférence le méthanol) afin d'obtenir l'ester correspondant à l'alcanol (de préférence méthylique).

L'ester obtenu est récupéré, à partir du milieu réactionnel par tout moyen connu : distillation ou cristallisation.

Le procédé de l'invention est particulièrement bien adapté à la préparation de l'acide D-2(4-hydroxyphénoxy)propionique.

L'exemple qui suit, illustre sa préparation. Il ne doit pas être considéré comme limitatif de l'invention.

Les abréviations utilisées ci-après, taux de transformation (TT), rendement (RR) et sélectivité (RT) ont la signification suivante :

$$TTHQ = \frac{\text{nombre de moles d'hydroquinone transformées}}{\text{nombre de moles d'hydroquinone introduites}} \text{ en \%}$$

$$RR_{HPPA/HQ} = \frac{\text{nombre de moles d'acide (4-hydroxyphénoxy)propionique formées}}{\text{nombre de moles d'hydroquinone introduites}} \text{ en \%}$$

$$RT_{HPPA/HQ} = \frac{\text{nombre de moles d'acide (4-hydroxyphénoxy)propionique formées}}{\text{nombre de moles d'hydroquinone transformées}} \text{ en \%}$$

$$RR_{HPPA/L\text{-}CPM} = \frac{\text{nombre de moles d'acide (4-hydroxyphénoxy)propionique formées}}{\text{nombre de moles d'ester méthylique de l'acide } \alpha\text{-chloropropionique introduites}} \text{ en \%}$$

$$\text{Pureté optique}_{L\text{-}CPM} = \frac{\text{L-CPM}}{\text{L - CPM + D - CPM}}$$

$$\text{Pureté optique}_{D\text{-}HPPA} = \frac{\text{D - HPPA}}{\text{D - HPPA + L - HPPA}}$$

Exemple 1

a- Préparation du sel de sodium de l'acide L-α-chloropropionique

Dans un ballon tricol 500 ml muni d'une ampoule de coulée, d'une agitation mécanique, on introduit 90,1 g de l'ester méthylique de l'acide L-α-chloropropionique (L-CPM) ayant une pureté optique de 97 %.

On coule en 30 minutes, 90 g d'une solution aqueuse de soude à 36 % en poids, en maintenant à 25-30°C, à l'aide d'un bain de saumure.

On laisse ensuite 1 heure à température ambiante.

On obtient un gel blanc que l'on solubilise par ajout de 20 g de méthanol.

b- Préparation de l'acide D-2(4-hydroxyphénoxy)propionique

Dans un réacteur équipé d'une double-enveloppe, muni d'une sonde thermométrique, d'une agitation mécanique, d'une ampoule de coulée, surmonté d'un réfrigérant et balayé par un courant d'azote, on charge en pied, 180 g de méthanol puis 88 g de soude caustique broyée ayant une pureté de 98 %, en maintenant la température à 30° C.

On charge par portions en 15 minutes, 121 g d'hydroquinone, sous azote et sous agitation à 350 tours/minute.

On porte le mélange à 45°C puis on ajoute 205 g de la solution obtenue selon a-, en 7h30.

On laisse encore 2 heures à 45°C sous agitation et sous azote.

On neutralise à un pH égal à 7 à l'aide de 157,5 g d'une solution aqueuse d'acide chlorhydrique à 37 %.

On solubilise le tout avec de l'eau déminéralisée et on dose par chromatographie liquide haute performance.

Les résultats obtenus sont les suivants :

- $TT_{HQ}$ = 58,2 %
- $RR_{HPPA/HQ}$ = 50,4 %
- $RT_{HPPA/HQ}$ = 86,6 %
- $RR_{HPPA/L\text{-}CPM}$ = 75,3%

$$\frac{\text{D - HPPA}}{\text{D - HPPA + L - HPPA}} = 96 \text{ \%}$$

Dans l'exemple comparatif qui suit, on prépare l'acide D-2(4-hydroxyphénoxy)propionique directement à partir du L-CPM, sans effectuer une saponification préalable de celui-ci.

Exemple comparatif a:

Préparation de l'acide D-2(4-hydroxyphénoxy)propionique

Dans un réacteur tel que décrit sous b-, on charge en pied 200 g de méthanol, 52,3 g d'eau, puis 121 g de soude broyée, par portions en régulant à 30°C, sous agitation à 350 tours/minute.

On ajoute par portions, 121 g d'hydroquinone en 15 mn, sous azote à 350 tours/minute.

On porte le mélange à 45°C, puis on effectue l'ajout de 90,1 g de L-CPM en 7 h 30 mn.

On laisse ensuite 2 heures à 45°C, sous agitation (350 tours/minute) et sous azote.

On refroidit le milieu à 20°C, puis on neutralise à un pH égal à 7 à l'aide de 160 g d'une solution aqueuse d'acide chlorhydrique à 37 %.

On solubilise le tout avec de l'eau déminéralisée et l'on dose par chromatographie liquide haute performance.

Les résultats obtenus sont les suivants :

- $TT_{HQ}$ = 51,4 %
- $RR_{HPPA/HQ}$ = 45,7 %
- $RT_{HPPA/HQ}$ = 89 %
- $RR_{HPPA/L\text{-}CPM}$ = 68,4 %

$$\frac{D\text{ - HPPA}}{D\text{ - HPPA} + L\text{ - HPPA}} = 91\ \%$$

Cet exemple comparatif montre l'importance d'effectuer la saponification préalable de l'ester méthylique de l'acide L-α-chloropropionique afin de conserver un bon rendement optique.

## Revendications

1. Procédé de préparation d'un acide α-(hydroxyphénoxy)alcanecarboxylique optiquement actif et de ses dérivés caractérisé par le fait qu'il consiste :

   - dans une première étape, à préparer une solution d'un sel alcalin d'un acide α-halogénoalcanecarboxylique optiquement actif, par saponification en milieu alcoolique, d'un ester alkylique d'un acide α-halogénoalcanecarboxylique optiquement actif à l'aide d'un hydroxyde de métal alcalin en solution aqueuse,
   - dans une deuxième étape, à engager directement la solution issue de l'étape précédente comprenant le sel alcalin d'un acide α-halogénoalcanecarboxylique dans la réaction avec un dihydroxybenzène ou un sel alcalin de dihydroxybenzène, en présence d'un hydroxyde de métal alcalin et dans un solvant alcoolique,
   - dans une dernière étape, à récupérer à partir du milieu réactionnel, l'acide α-(hydroxyphénoxy)alcanecarboxylique optiquement actif ou dérivé issu de celui-ci.

2. Procédé selon la revendication 1 caractérisé par le fait que l'ester alkylique d'un acide α-halogénoalcanecarboxylique répond à la formule (II) :

$$X\text{ - }A\text{ - }COO\ R \qquad \text{(II)}$$

   dans ladite formule (II) :

   - A représente un radical méthylène substitué par 1 ou 2 groupements alkyle ayant de 1 à 4 atomes de carbone, de préférence un groupement méthyle,
   - R représente un groupement alkyle, linéaire ou ramifié ayant de 1 à 4 atomes de carbone,
   - X représente un atome d'halogène, de préférence un atome de chlore ou de brome.

3. Procédé selon l'une des revendications 1 et 2 caractérisé par le fait que le composé de formule (II) est un ester, de préférence, méthylique ou éthylique des acides carboxyliques suivants sous forme D ou L :

- l'acide α-chloropropionique
- l'acide α-bromopropionique
- l'acide α-chlorobutyrique
- l'acide α-bromobutyrique
- l'acide α-chloroisobutyrique
- l'acide α-bromoisobutyrique

**4.** Procédé selon l'une des revendications 1 à 3 caractérisé par le fait que l'agent de saponification est l'hydroxyde de sodium ou de potassium, de préférence l'hydroxyde de sodium, mise en oeuvre sous forme de solution aqueuse dont la concentration varie avantageusement entre 30 et 60 % en poids.

**5.** Procédé selon la revendication 4 caractérisé par le fait que la quantité de l'agent de saponification est telle que le rapport entre le nombre de moles d'hydroxyde de métal alcalin et le nombre de moles de l'ester alkylique de l'acide α-halogénoalcanecarboxylique varie entre 1,0 et 1,2.

**6.** Procédé selon l'une des revendications 1 à 5 caractérisé par le fait que l'alcool qui est utilisé comme solvant réactionnel est de même nature que l'alcool libéré par saponification de l'ester.

**7.** Procédé selon la revendication 6 caractérisé par le fait que l'alcool utilisé est le méthanol ou l'éthanol.

**8.** Procédé selon l'une des revendications 6 et 7 caractérisé par le fait que la quantité d'alcool mise en jeu est déterminée de telle sorte qu'elle représente par rapport au poids du sel de l'acide obtenu de 5 à 40 %.

**9.** Procédé selon l'une des revendications 1 à 8 caractérisé par le fait que la température de la réaction de saponification est choisie, de préférence entre 0°C et 40°C et plus préférentiellement entre 20°C et 25°C.

**10.** Procédé selon l'une des revendications 1 à 9 caractérisé par le fait qu'il consiste dans une première étape, à introduire la solution d'hydroxyde de métal alcalin dans le milieu réactionnel comprenant l'ester alkylique de l'acide α-halogénoalcanecarboxylique : l'addition étant faite de manière progressive, en continu ou en discontinu, par fractions et par le fait qu'il consiste à ajouter, en début ou en fin de saponification le solvant alcoolique.

**11.** Procédé selon l'une des revendications 1 à 10 caractérisé par le fait que le dihydroxybenzène répond à la formule (III):

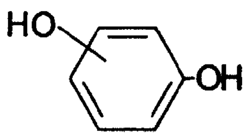

**(III)**

dans ladite formule (III) le groupement hydroxyle étant en position 2-, 3- ou 4- par rapport au groupement hydroxyle et est de préférence, l'hydroquinone.

**12.** Procédé selon la revendication 11 caractérisé par le fait que l'on salifie le dihydroxybenzène par un hydroxyde de métal alcalin, de préférence l'hydroxyde de sodium mis en oeuvre en une quantité telle que le rapport entre le nombre de moles d'hydroxyde de métal alcalin et le nombre de moles de dihydroxybenzène varie, de préférence, entre 2,0 et 2,2.

**13.** Procédé selon l'une des revendications 1 à 12 caractérisé par le fait que la réaction de salification est conduite en milieu alcoolique, de préférence le méthanol ou l'éthanol.

**14.** Procédé selon la revendication 13 caractérisé par le fait que la quantité d'alcool mise en jeu est déterminée de telle sorte qu'elle représente par rapport au poids du dihydroxybenzène engagé de 100 à 300 %, de préférence, environ 150 %.

**15.** Procédé selon l'une des revendications 11 à 14 caractérisé par le fait que la température de la réaction de salification est choisie, de préférence entre 20°C et 60°C.

16. Procédé selon l'une des revendications 11 à 15 caractérisé par le fait qu'il consiste à introduire l'hydroxyde de métal alcalin dans le milieu réactionnel comprenant le dihydroxybenzène et le solvant alcoolique: l'addition étant faite de manière progressive, en continu ou en discontinu, par fractions.

17. Procédé selon l'une des revendications 1 à 16 caractérisé par le fait que la quantité de dihydroxybenzène salifié et du sel alcalin d'un acide α-halogénoalcanecarboxylique issue de l'étape précédente est déterminée de telle sorte que le rapport entre le nombre de moles de dihydroxybenzène salifié et le nombre de moles du sel alcalin d'un acide α-halogénoalcanecarboxylique est compris avantageusement entre 1,0 et 1,5.

18. Procédé selon l'une des revendications 1 à 17 caractérisé par le fait qu'il consiste à introduire progressivement la solution issue de l'étape précédente comprenant le sel alcalin d'un acide α-halogénoalcanecarboxylique dans le milieu réactionnel comprenant le dihydroxybenzène sous forme salifiée.

19. Procédé selon la revendication 18 caractérisé par le fait que la température de la réaction est choisie, de préférence inférieure à 60°C, plus particulièrement entre 30°C et 55°C et encore plus préférentiellement entre 40°C et 45°C.

20. Procédé selon l'une des revendications 1 à 19 caractérisé par le fait qu'il consiste dans une dernière étape, à récupérer à partir du milieu réactionnel, l'acide α-(hydroxyphénoxy)alcanecarboxylique optiquement actif, par neutralisation à un pH égal à environ 7,0 par ajout d'un acide fort, de préférence l'acide chlorhydrique ; suivie d'une distillation ; d'une extraction du dihydroxybenzène en excès par un solvant organique, de préférence, un solvant acétonique et encore plus préférentiellement, la méthylisobutylcétone ou un solvant de type éther; d'une séparation des phases organique et aqueuse ; d'une distillation de la solution aqueuse résiduaire de façon à éliminer le solvant organique; puis d'une acidification à un pH inférieur ou égal à 1 de façon à précipiter l'acide α-(hydroxyphénoxy) alcanecarboxylique.

21. Procédé selon l'une des revendications 1 à 20 caractérisé par le fait qu'i consiste à préparer un ester alkylique d'un acide α-(hydroxyphénoxy)alcanecarboxylique optiquement actif, à partir d'un acide α-(hydroxyphénoxy)alcanecarboxylique obtenu selon l'une des revendications 1 à 20, par neutralisation de la phase aqueuse à un pH inférieur ou égal à 2,0 par ajout d'un acide fort, de préférence l'acide chlorhydrique ; puis chauffage sous reflux de l'alcanol (de préférence le méthanol) afin d'obtenir l'ester correspondant à l'alcanol (de préférence méthylique) ; puis récupération de l'ester désiré.

22. Utilisation du procédé décrit dans l'une des revendications 1 à 21 pour préparer l'acide D-2(4-hydroxyphénoxy) propionique et ses dérivés, de préférence, ses esters alkyliques ayant de 1 à 4 atomes de carbone.

**Claims**

1. A process for the preparation of an optically active α-(hydroxyphenoxy)-alkanecarboxylic acid, characterised in that it consists of:

   - in a first step, preparing a solution of an alkaline salt of an optically active a-halogeno-alkanecarboxylic acid by saponification, in an alcoholic medium, of an alkyl ester of an optically active α-halogeno-alkanecarboxylic acid using an alkali metal hydroxide in aqueous solution,
   - in a second step, directly using the solution from the preceding step, comprising the alkaline salt of the α-halogeno-alkanecarboxylic acid, in a reaction with a dihydroxybenzene or an alkaline salt of a dihydroxybenzene, in the presence of an alkali metal hydroxide and in an alcoholic solvent,
   - in a final step, recovering the optically active α-(hydroxyphenoxy)-alkanecarboxylic acid or derivative thereof from the reaction medium.

2. A process according to claim 1, characterised in that the alkyl ester of the α-halogeno-alkanecarbOxylic acid has formula (II):

$$X\text{-}A\text{-}COOR \qquad (II)$$

where:

- A represents a methylene radical which is substituted with 1 or 2 alkyl groups containing 1 to 4 carbon atoms, preferably a methyl group,
- R represents a linear or branched alkyl group containing 1 to 4 carbon atoms,
- X represents a halogen atom, preferably a chlorine or bromine atom.

**3.** A process according to claim 1 or claim 2, characterised in that the compound with formula (II) is an ester, preferably a methyl or ethyl ester of the following carboxylic acids in their D or L form:

- α-chloropropionic acid,
- α-bromopropionic acid,
- α-chlorobutyric acid,
- α-bromobutyric acid,
- α-chloroisobutyric acid,
- α-bromoisobutyric acid.

**4.** A process according to any one of claims 1 to 3, characterised in that the saponification agent is sodium or potassium hydroxide, preferably sodium hydroxide, used in the form of an aqueous solution, advantageously with a concentration of between 30% and 60% by weight.

**5.** A process according to claim 4, characterised in that the quantity of saponification agent is such that the ratio between the number of moles of alkali metal hydroxide and the number of moles of the alkyl ester of the α - halogeno-alkanecarboxylic acid is between 1.0 and 1.2.

**6.** A process according to any one of claims 1 to 5, characterised in that the alcohol which is used as the reaction solvent is the same as the alcohol liberated during saponification of the ester.

**7.** A process according to claim 6, characterised in that the alcohol used is methanol or ethanol.

**8.** A process according to claim 6 or claim 7, characterised in that the quantity of alcohol used is such that it represents 5% to 40% of the weight of the salt of the acid obtained.

**9.** A process according to any one of claims 1 to 8, characterised in that the saponification reaction temperature is preferably between 0°C and 40°C, most preferably between 20°C and 25°C.

**10.** A process according to any one of claims 1 to 9, characterised in that it consists of a first step of introducing the alkali metal hydroxide solution into the reaction medium comprising the alkyl ester of the α-halogeno-alkanecarboxylic acid; addition being carried out gradually, in fractions, continuously or discontinuously, and in that the alcoholic solvent is added at the beginning or end of saponification.

**11.** A process according to any one of claims I to 10, characterised in that the dihydroxybenzene has formula (III):

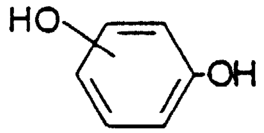

(III)

where the hydroxy group is in the 2-, 3- or 4- position with respect to the hydroxy group and is preferably hydroquinone.

**12.** A process according to claim 11, characterised in that the dihydroxybenzene is salified with an alkali metal hydroxide, preferably sodium hydroxide, in a quantity such that the ratio between the number of moles of alkali metal hydroxide and the number of moles of dihydroxybenzene is preferably between 2.0 and 2.2.

**13.** A process according to any one of claims 1 to 12, characterised in that the salification reaction is conducted in an alcoholic medium, preferably methanol or ethanol.

**14.** A process according to claim 13, characterised in that the quantity of alcohol used is such that it represents 100% to 300%, preferably about 150%, of the weight of the dihydroxybenzene used.

**15.** A process according to any one of claims 11 to 14, characterised in that the salification reaction temperature is preferably between 20°C and 60°C.

**16.** A process according to any one of claims 11 to 15, characterised in that it consists of introducing the alkali metal hydroxide into the reaction medium comprising the dihydroxybenzene and the alcoholic solvent: addition being made gradually, in fractions, continuously or discontinuously.

**17.** A process according to any one of claims 1 to 16, characterised in that the quantity of salified dihydroxybenzene and the alkaline salt of the α-halogeno-alkanecarboxylic acid from the preceding step is such that the ratio between the number of moles of salified dihydroxybenzene and the number of moles of the alkaline salt of the α-halogeno-alkanecarboxylic acid is advantageously between 1.0 and 1.5.

**18.** A process according to any one of claims 1 to 17, characterised in that it consists of gradually introducing the solution from the preceding step, comprising the alkaline salt of the α-halogeno-alkanecarboxylic acid, into the reaction medium comprising the dihydroxybenzene in the form of its salt.

**19.** A process according to claim 18, characterised in that the reaction temperature is preferably less than 60°C, more particularly between 30°C and 55°C and most preferably between 40°C and 45°C.

**20.** A process according to any one of claims 1 to 19, characterised in that it consists, in a final step, of recovering the optically active α-(hydroxyphenoxy)-alkanecarboxylic acid from the reaction medium, by neutralising to a pH of about 7.0 by addition of a strong acid, preferably hydrochloric acid; followed by distillation; extracting the excess dihydroxybenzene using an organic solvent, preferably a ketone, more preferably methylisobutyl ketone or an ether type solvent; separating the organic and aqueous phases; distilling the residual aqueous solution to eliminate the organic solvent; then acidifying to a pH of 1 or less to precipitate the α-(hydroxyphenoxy)-alkanecarboxylic acid.

**21.** A process according to any one of claims 1 to 20, characterised in that it consists of preparing an alkyl ester of an optically active α-(hydroxyphenoxy)-alkanecarboxylic acid from the α-(hydroxyphenoxy)-alkanecarboxylic acid obtained in accordance with any one of claims 1 to 20, by neutralising the aqueous phase to a pH of 2.0 or less by addition of a strong acid, preferably hydrochloric acid; then heating to reflux the alkanol (preferably methanol) to obtain the ester corresponding to the alkanol (preferably the methyl ester); then recovering the desired ester.

**22.** The use of a process as defined in any one of claims 1 to 21 to prepare D-2-(4-hydroxyphenoxy)-propionic acid and its derivatives, preferably alkyl esters containing 1 to 4 carbon atoms.

**Patentansprüche**

**1.** Verfahren zur Herstellung einer optisch aktiven α-(Hydroxyphenoxy)alkancarbonsäure und ihrer Derivate, dadurch gekennzeichnet, daß

- man in einem ersten Verfahrensschritt eine Lösung eines Alkalisalzes einer optisch aktiven α-Halogenalkancarbonsäure durch Verseifung in alkoholischem Milieu eines Alkylesters der optisch aktiven α-Halogenalkancarbonsäure mit Hilfe eines Alkalimetallhydroxids in wäßriger Lösung herstellt,
- man in einem zweiten Verfahrensschritt die aus dem vorangehenden Verfahrensschritt stammende Lösung, welche das Alkalimetallsalz der α-Halogenalkancarbonsäure umfaßt, direkt bei der Reaktion mit einem Dihydroxybenzol oder einem Alkalisalz des Dihydroxybenzols in Gegenwart eines Alkalimetallhydroxids und in einem alkoholischen Lösungsmittel einsetzt und
- man in einem letzten Verfahrensschritt die optisch aktive α-(Hydroxyphenoxy)alkancarbonsäure oder ihr Derivat aus dem Reaktionsmilieu abtrennt.

**2.** Verfahren nach Anspruch 1, dadurch gekennzeichnet, daß der Alkylester der α-Halogenalkancarbonsäure der Formel (II)

X-A-COOR (II)

entspricht, in der:

- A einen mit 1 oder 2 Alkylgruppen mit 1 bis 4 Kohlenstoffatomen, vorzugsweise einer Methylgruppe, substituierten Methylenrest darstellt,
- R eine lineare oder verzweigte Alkylgruppe mit 1 bis 4 Kohlenstoffatomen darstellt und
- X ein Halogenatom, vorzugsweise ein Chlor- oder Bromatom, darstellt.

**3.** Verfahren nach Anspruch 1 oder 2, dadurch gekennzeichnet, daß die Verbindung der Formel (II) ein Ester, vorzugsweise ein Methyl- oder Ethylester der folgenden Carbonsäuren entweder in der D-Form oder L-Form ist:

- α-Chlorpropionsäure
- α-Brompropionsäure
- α-Chlorbuttersäure
- α-Brombuttersäure
- α-Chlorisobuttersäure
- α-Bromisobuttersäure.

**4.** Verfahren nach einem der Ansprüche 1 bis 3, dadurch gekennzeichnet, daß das Verseifungsreagenz Natrium- oder Kaliumhydroxid, vorzugsweise Natriumhydroxid, ist, welche in Form einer wäßrigen Lösung eingesetzt werden, deren Konzentration vorzugsweise zwischen 30 und 60 Gew.-% variiert.

**5.** Verfahren nach Anspruch 4, dadurch gekennzeichnet, daß die Menge an Verseifungsreagenz derart ist, daß das Verhältnis zwischen der Anzahl der Mol an Alkalimetallhydroxid und der Anzahl an Mol an Alkylester der α-Halogenalkancarbonsäure zwischen 1,0 und 1,2 variiert.

**6.** Verfahren nach einem der Ansprüche 1 bis 5, dadurch gekennzeichnet, daß der Alkohol, welcher als Reaktionslösungsmittel verwendet wird, von derselben Natur ist wie der Alkohol, der bei der Verseifung des Esters freigesetzt wird.

**7.** Verfahren nach Anspruch 6, dadurch gekennzeichnet, daß der verwendete Alkohol Methanol oder Ethanol ist.

**8.** Verfahren nach Anspruch 6 oder 7, dadurch gekennzeichnet, daß die eingesetzte Alkoholmenge derart festgelegt wird, daß sie, bezogen auf das Gewicht des erhaltenen Säuresalzes, 5 bis 40 % darstellt.

**9.** Verfahren nach einem der Ansprüche 1 bis 8, dadurch gekennzeichnet, daß die Reaktionstemperatur für die Verseifung vorzugsweise zwischen 0 °C und 40 °C, insbesondere zwischen 20 °C und 25 °C, ausgewählt ist.

**10.** Verfahren nach einem der Ansprüche 1 bis 9, dadurch gekennzeichnet, daß man in einem ersten Verfahrensschritt die Alkalimetallhydroxidlösung in das Reaktionsmilieu einbringt, welches den Alkylester der α-Halogenalkancarbonsäure umfaßt, wobei die Zugabe allmählich, kontinuierlich oder diskontinuierlich, fraktionsweise erfolgt und daß man zu Beginn oder zum Ende der Verseifung das alkoholische Lösungsmittel hinzugibt.

**11.** Verfahren nach einem der Ansprüche 1 bis 10, dadurch gekennzeichnet, daß das Dihydroxybenzol der Formel (III)

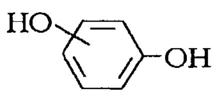

(III)

entspricht, wobei sich in der Formel (III) die Hydroxylgruppe in 2-, 3- oder 4-Position in bezug auf die Hydroxylgruppe befindet, und vorzugsweise Hydrochinon ist.

**12.** Verfahren nach Anspruch 11, dadurch gekennzeichnet, daß man das Dihydroxybenzol durch ein Alkalimetallhydroxid, vorzugsweise Natriumhydroxid, versalzt, das in einer solchen Menge eingesetzt wird, daß das Verhältnis zwischen der Anzahl an Mol Alkalimetallhydroxid und der Anzahl an Mol Dihydroxybenzol vorzugsweise zwischen

2,0 und 2,2 variiert.

**13.** Verfahren nach einem der Ansprüche 1 bis 12, dadurch gekennzeichnet, daß die Versalzungsreaktion in alkoholischem Milieu, vorzugsweise in Methanol oder Ethanol, durchgeführt wird.

**14.** Verfahren nach Anspruch 13, dadurch gekennzeichnet, daß die eingesetzte Alkoholmenge derart festgelegt wird, daß sie, bezogen auf das Gewicht des eingesetzten Dihydroxybenzols, 100 bis 300 %, vorzugsweise etwa 150 %, darstellt.

**15.** Verfahren nach einem der Ansprüche 11 bis 14, dadurch gekennzeichnet, daß die Reaktionstemperatur für die Versalzung vorzugsweise zwischen 20 °C und 60 °C ausgewählt ist.

**16.** Verfahren nach einem der Ansprüche 11 bis 15, dadurch gekennzeichnet, daß man das Alkalimetallhydroxid in das Reaktionsmilieu, welches das Dihydroxybenzol und das alkoholische Lösungsmittel umfaßt, einträgt, wobei die Zugabe schrittweise, kontinuierlich oder diskontinuierlich, in Fraktionen erfolgt.

**17.** Verfahren nach einem der Ansprüche 1 bis 16, dadurch gekennzeichnet, daß die Menge an versalztem Dihydroxybenzol und an Alkalisalz einer $\alpha$-Halogenalkancarbonsäure, welche aus dem vorhergehenden Verfahrensschritt stammt, derart festgelegt wird, daß das Verhältnis zwischen der Anzahl an Mol versalztem Dihydroxybenzol und der Anzahl an Mol Alkalisalz der $\alpha$-Halogenalkancarbonsäure vorzugsweise zwischen 1,0 und 1,5 liegt.

**18.** Verfahren nach einem der Ansprüche 1 bis 17, dadurch gekennzeichnet, daß man die aus dem vorhergehenden Verfahrensschritt stammende Lösung, die das Alkalisalz einer $\alpha$-Halogenalkancarbonsäure enthält, dem Reaktionsmilieu schrittweise zusetzt, welches das Dihydroxybenzol in versalzter Form enthält.

**19.** Verfahren nach Anspruch 18, dadurch gekennzeichnet, daß die Reaktionstemperatur vorzugsweise unterhalb von 60 °C, insbesondere zwischen 30 °C und 55 °C, insbesondere zwischen 40 °C und 45 °C, ausgewählt ist.

**20.** Verfahren nach einem der Ansprüche 1 bis 19, dadurch gekennzeichnet, daß man in einem letzten Verfahrensschritt die optisch aktive $\alpha$-(Hydroxyphenoxy)alkancarbonsäure durch Neutralisation auf einen pH-Wert von etwa 7,0 durch Zugabe einer starken Säure, vorzugsweise Salzsäure, aus dem Reaktionsmilieu isoliert, woran sich eine Destillation, eine Extraktion des überschüssigen Dehydroxybenzols mit einem organischen Lösungsmittel, vorzugsweise einem Acetonlösungsmittel, insbesondere Methylisobutylketon, oder einem Lösungsmittel vom Ethertyp, sowie eine Trennung der organischen und wäßrigen Phase und eine Destillation der restlichen wäßrigen Lösung anschließt, so daß das organische Lösungsmittel entfernt wird, gefolgt von einer Ansäuerung auf einen pH-Wert von weniger oder gleich 1, so daß die $\alpha$-(Hydroxyphenoxy)alkancarbonsäure ausfällt.

**21.** Verfahren nach einem der Ansprüche 1 bis 20, dadurch gekennzeichnet, daß man einen optisch aktiven Alkylester einer $\alpha$-(Hydroxyphenoxy)alkancarbonsäure ausgehend von einer $\alpha$-(Hydroxyphenoxy)alkancarbonsäure, die nach einem der Ansprüche 1 bis 20 erhalten worden ist, durch Neutralisation der wäßrigen Phase auf einen pH-Wert von weniger oder gleich 2,0 durch Zugabe einer starken Säure, vorzugsweise Salzsäure, herstellt mit anschließendem Erhitzen des Alkanols (vorzugsweise Methanol) unter Rückfluß, um den dem Alkanol entsprechenden Ester (vorzugsweise den Methylester) zu erhalten, und anschließender Isolierung des gewünschten Esters.

**22.** Anwendung des in einem der Ansprüche 1 bis 21 beschriebenen Verfahrens zur Herstellung von D-2(4-Hydroxyphenoxy)propionsäure und ihren Derivaten, vorzugsweise ihren Alkylestem mit 1 bis 4 Kohlenstoffatomen.